# FASCICULE DE BREVET EUROPEEN

(11) **EP 4 256 390 B1**
(45) Date de publication et mention de la délivrance du brevet: **02.12.2026**
(21) Numéro de dépôt: 21835192.2
(22) Date de dépôt: 02.12.2021
(51) Int. Cl.: G02B 27/00, G06V 40/16, A61B 5/16, G02B 27/01, A61B 5/00, G06F 3/01, G16H 20/70, G06V 20/20

(54) **DISPOSITIF IMMERSIF**
IMMERSIVE VORRICHTUNG
IMMERSIVE DEVICE

(30) Priorité: 04.12.2020 FR 2012731
(43) Date de publication de la demande: 11.10.2023
(73) Titulaire: DREAMSENS, 26320 Saint-Marcel-lès-Valence (FR)
(72) Inventeur: GRICOURT, Thierry, 26300 Bourg de Peage (FR); ALLÉLY, Mathias Guy, 38260 Gillonnay (FR); LAURENS, Paul Jacques, 38260 Pommier de Beaurepaire (FR)
(74) Mandataire: Fidal Innovation
(86) Numéro de dépôt international: PCT/EP2021/084079
(87) Numéro de publication internationale: WO 2022/117776

(56) Documents cités:
- EP-A1- 3 779 559
- WO-A1-2017/006872
- WO-A1-2018/227424
- WO-A1-2019/246576
- WO-A1-2020/088102
- WO-A2-2020/039152
- US-A1- 2017 323 485
- US-A1- 2018 303 397
- US-A1- 2020 139 077
- US-B1- 10 657 718

## Description

### DOMAINE DE L'INVENTION

La présente invention se rapporte à un dispositif immersif.

### ARRIERE-PLAN TECHNOLOGIQUE

Plus précisément, l'invention se rapporte à un casque audio-visuel immersif utilisable comme source de bien-être et thérapeutique.

Dans le domaine des dispositifs de réalité virtuelle, il est connu des lunettes qui proposent une image à chaque œil et qui sont dotées de capteurs gyroscopiques pour garder l'orientation d'une image malgré les mouvements de la tête. Le fait que l'image garde la même orientation malgré une inclinaison de la tête donne un effet de réalité virtuelle.

L'image envoyée à l'œil droit et à l'œil gauche peuvent avoir été filmées selon deux angles de vue légèrement différents, reproduisant le même écartement entre deux yeux d'humain. Ainsi en projetant ces deux images gauche / droite respectivement face aux yeux gauche et droit de l'utilisateur, on obtient un effet de profondeur en trois dimensions.

De plus, la portion de l'image vidéo projetée dans les lunettes est décalée en fonction du mouvement de la tête détecté grâce à des capteurs quand elle se tourne d'un côté, la partie affichée devant l'œil est décalée proportionnellement au mouvement dans le sens opposé.

Avec une image vidéo 3D vectorisée, on peut simuler des déplacements avec une image projetée qui va modifier la disposition relative des éléments qui composent une image en fonction de la détection de mouvements de l'utilisateur grâce à des capteurs selon les règles de la perspective afin de reproduire les changements de points de vue, comme dans la réalité.

Tous ces procédés combinés permettent de générer une réalité virtuelle pour un utilisateur. De telles lunettes sont connues par exemple d'US2017/323,485 ou US 10,657,718. À la place de lunettes, il est connu des visières couvrant le visage ou des casques offrant une surface à distance du visage formant un écran incurvé sur lequel on peut projeter des images. Dans de tels dispositifs, l'effet 3D est obtenu grâce à une distance suffisante entre l'œil et le point de l'écran le plus proche de l'œil, comme le précise le document WO2009066408A1. De tels casques de réalité virtuelle sont mentionnés dans WO 2019/246,576 ou WO 2020/088,102.

En effet, le sens de la profondeur du sens visuel humain a été considéré comme étant le plus affecté par la parallaxe binoculaire. Autrement dit, différentes images entre les deux yeux sont générées par la vue d'un être humain et la parallaxe binoculaire permet la perception du sens de la profondeur. Mais l'expérience montre qu'une image 2D projetée à une certaine distance de l'œil humain, au-delà de 21,7 cm, sur un écran galbé, par exemple sphérique, peut être interprétée par un cerveau humain comme une image 3D.

L'image projetée peut également varier en fonction de la position et des mouvements de l'utilisateur.

US 2020/139,077 semble plutôt décrire une tablette sur laquelle est affichée une image en réalité virtuelle.

Il existe par ailleurs des dispositifs de reconnaissance des émotions à partir de l'image d'un visage : les dix émotions que sont la joie, la tranquillité, la tristesse, la colère, la peur, la surprise, l'ennui, le mépris, la fatigue et le dégoût peuvent être reconnues. Ce sont des dispositifs qui sont fréquemment utilisés dans le domaine du marketing et de la vente au grand public pour proposer une expérience client plus personnalisée.

L'invention vise à proposer un dispositif spécialement adapté à des traitements médicaux qui combine les deux fonctions de réalité virtuelle et de reconnaissance des émotions en permettant d'adapter les stimulations fournies en réalité virtuelle en temps réel aux émotions exprimées sur le visage d'un utilisateur, tout en étant le moins invasif possible.

Afin que ledit dispositif puisse être utilisé dans le cadre d'une thérapie médicale, il est fondamental qu'il soit le moins invasif possible pour éviter qu'il ne soit source d'angoisse, de stress et de fatigue pour l'utilisateur.

Ainsi, le cerveau de l'utilisateur est stimulé de façon adaptée pendant autant de temps que nécessaire au cours d'une journée, permettant ainsi de lutter contre l'ennui, la tristesse et la peur et de retarder la dégénérescence des fonctions cognitives du cerveau de l'utilisateur.

L'application thérapeutique visée par l'invention est celle des maladies neurodégénératives, plus précisément celle de la maladie d'Alzheimer, de Parkinson ou apparentées. Des appareils conventionnels sont également connus dans les documents US 2017/323485 et WO 2018/22742.

### RESUME DE L'INVENTION

Ainsi, l'invention est définie dans les revendications et se rapporte à un dispositif immersif qui comprend :
- un élément de fixation pour fixer le dispositif sur la tête d'un utilisateur,
- un système de reconnaissance d'émotions apte à détecter des émotions de l'utilisateur, le système de reconnaissance d'émotions comprenant au moins un capteur et un calculateur analysant un ou plusieurs signaux du capteur pour indiquer une émotion choisie parmi la joie, la tranquillité, la tristesse, la colère, la peur, la surprise, l'ennui, le mépris, la fatigue et le dégoût,
- un système de diffusion de séquences vidéo, une séquence vidéo comprenant une suite d'images diffusées, le système de diffusion étant adapté pour sélectionner une séquence vidéo classée par rapport à au moins une émotion de l'utilisateur communiquée par le calculateur du système de détection, et pour diffuser celle-ci sur un écran,
- un système d'observation pour capter, diffuser et/ou enregistrer les réactions de l'utilisateur.

Par immersif, on entend un dispositif qui permet à l'utilisateur de se déconnecter de son environnement et de lui donner l'impression d'être plongé dans un environnement différent en sollicitant ses sens suivants : la vue et l'ouïe.

Le système de diffusion de séquences vidéo utilisé peut être plus ou moins sophistiqué, et peut comprendre de multiples capteurs pour déterminer des positions et des mouvements de l'utilisateur, en vue de reproduire une réalité virtuelle en fonction de ces positions et mouvements de l'utilisateur. Alternativement, dans un souci de limitation du poids et du coût du dispositif, le système de diffusion de séquences vidéo utilisé peut être un simple diffuseur de séquences vidéo sans tenir compte des mouvements ou de la position de l'utilisateur au moment de la diffusion.

Les vidéos peuvent inclure du son diffusé de façon synchronisée avec la diffusion des images. La diffusion peut se faire par haut-parleurs intégrés au dispositif ou extérieurs.

Au final, le meilleur compromis, en fonction de l'application, entre poids et performance, est recherché.

Les vidéos diffusées peuvent être de 3 catégories :
- Détente, plaisir, divertissement ;
- Réminiscence, attention, mémoire, raisonnement, maitrise de l'espace ;
- Mouvement, interaction, motivation.

Le système de diffusion de séquences vidéo est piloté par un programme d'ordinateur qui sélectionne la vidéo adaptée selon certains critères, comme la fréquence de diffusion, la notation de la vidéo, ou bien un choix aléatoire. La sélection se fait parmi une liste de séquences vidéo stockée sous forme de fichiers informatiques vidéos réunis sur une mémoire.

Le système de diffusion de séquences vidéo est alimenté en électricité par une source d'énergie électrique, par exemple extérieure, via une connexion filaire.

Par vidéo, au sens de l'invention, on entend une séquence d'une suite d'images diffusées, par exemple de quelques minutes au moins, dont le contenu a été testé comme adapté à une ou plusieurs émotions humaines dans un but de générer de la joie, de la tranquillité ou de stimuler les fonctions cognitives et la concentration du cerveau de l'utilisateur.

Le système de diffusion de séquences vidéo selon un procédé de réalité virtuelle, bien connu de l'Homme du métier, peut intégrer des capteurs de mouvement et de position de l'utilisateur. Ces informations permettent d'ajuster la vidéo diffusée afin de simuler une scène extérieure en trois dimensions. Le système de diffusion de séquences vidéo peut gérer tous types de contenus vidéo, depuis une image enregistrée purement 2D à une image enregistrée et projetée sur 360°, correspondant à un angle solide de 4 fois Pi stéradians, ou une succession de telles images.

Par exemple, le personnel médical peut surveiller la capacité de l'utilisateur à réagir aux vidéos diffusées grâce au système d'observation. On dit alors que le système d'observation est adapté pour diffuser les réactions de l'utilisateur au personnel médical.

Le dispositif immersif qui vient d'être présenté est caractérisé en ce qu'il comprend une visière qui sert d'écran, et s'étendant depuis au-dessus du haut du front jusqu'au niveau face au menton, et un lien entre l'élément de fixation et la visière.

Selon différents aspects, il est possible de prévoir l'une et/ou l'autre des dispositions ci-dessous.

Le système de détection des émotions traduit et transmet en temps réel au système de diffusion de vidéos les émotions détectées, éventuellement pondérées. Ainsi, le système de diffusion de vidéos peut sélectionner une vidéo à diffuser en fonction de la ou des émotions détectées.

Les émotions détectables sont une des émotions ou une combinaison des émotions suivantes : la joie, la tranquillité, la tristesse, la colère, la peur, la surprise, l'ennui, le mépris, la fatigue et le dégoût. Chaque émotion peut également être pondérée en fonction d'un critère d'intensité.

Le système de détection intègre ainsi le traitement du signal représentatif des émotions de l'utilisateur, bien connu de l'Homme du métier, et communique cette information au système de diffusion de vidéos afin de sélectionner une vidéo adaptée. En fonction du niveau de la persistance de l'émotion, une vidéo déjà en train d'être diffusée peut être interrompue par le système de détection. Sinon, c'est l'émotion détectée à la fin d'une vidéo qui est prise en compte pour faire le choix de la catégorie de la prochaine vidéo à diffuser.

Le système de diffusion enregistre la liste des émotions détectées de façon horodatée, ainsi que l'intensité et la durée de chaque émotion. Cette liste est rendue accessible à des fins d'analyse au personnel médical.

Grâce à ces dispositions, un utilisateur, atteint par exemple de la maladie d'Alzheimer, peut être stimulé spécifiquement en fonction de ses émotions par le biais de vidéos dans le cadre d'une thérapie mise au point par les médecins traitants. De telles stimulations ont pour but de solliciter certaines fonctions du cerveau afin de retarder, voire d'éviter leur dégénérescence.

Selon une réalisation, le système d'observation comprend une caméra, orientée vers le visage de l'utilisateur.

Ainsi on ne gêne pas la visibilité des images diffusées pour l'utilisateur.

Selon une réalisation, l'élément de fixation est ajustable.

Ainsi, on peut rendre le port du dispositif plus confortable et compatible avec tous les utilisateurs.

Le système de diffusion de séquences vidéo comprend en outre un vidéoprojecteur.

Ainsi on peut utiliser une vidéo source 2D et, à condition de positionner l'écran à environ 20 cm des yeux de l'utilisateur, on obtient un effet 3D.

Selon une réalisation, le système de diffusion de séquences vidéo comprend un support permettant de supporter le vidéoprojecteur de façon réversible.

Ainsi, on limite l'aspect invasif propre à ce type de dispositif. On améliore également l'hygiène en évitant les contacts avec le visage.

Enfin, on permet le changement du système de diffusion de vidéos en cas de panne, ou d'amélioration technologique au niveau du projecteur très simplement.

L'écran est une partie d'une visière galbée pour être à distance du visage en position d'utilisation et pour occuper le champ visuel de l'utilisateur.

En limitant ainsi les points de contact entre le dispositif et le visage de l'utilisateur, on limite l'aspect invasif propre à ce type de dispositif.

Sur le plan de l'hygiène, on réduit ainsi également d'autant les risques de contamination par des virus et des bactéries.

Le dispositif est ainsi adapté à des personnes faibles physiquement et/ou psychologiquement.

Le support adapté à un vidéoprojecteur est fixé sur une partie dudit dispositif destinée à être au-dessus de la tête de l'utilisateur en position d'utilisation.

La chaleur émise par le vidéoprojecteur s'échappe ainsi au-dessus de la tête de l'utilisateur. Le centre de gravité du dispositif est ainsi plus proche de l'axe du corps de l'utilisateur en position debout ou assise. Ainsi, on limite l'aspect invasif propre à ce type de dispositif.

Selon une réalisation, le dispositif immersif est doté dans sa partie basse d'une bavette.

Le dispositif immersif est un dispositif thérapeutique ou de bien-être.

Selon une réalisation, le dispositif est adapté pour déconnecter l'utilisateur de son environnement et lui donner l'impression d'être plongé dans un environnement différent en sollicitant ses sens : la vue et l'ouïe.

Selon une réalisation, les séquences vidéo comprennent chacune une suite d'images diffusées dont le contenu a été testé comme adapté à une ou plusieurs émotions humaines dans un but de générer de la joie, de la tranquillité ou de stimuler les fonctions cognitives et la concentration du cerveau de l'utilisateur.

Selon une réalisation, le dispositif immersif ne présente aucun point de contact avec le visage en position d'utilisation.

Selon une réalisation, le lien rigide est évasé vers l'extérieur dans le sens allant de l'élément de fixation vers la visière.

Selon une réalisation, la visière présente un pourtour périphérique distant du visage de l'utilisateur en position d'utilisation.

Selon un autre aspect, on prévoit un système comprenant un poste de contrôle et au moins un voire plusieurs, dispositifs immersifs.

Ainsi on renforce la solidité du dispositif et on facilite sa manipulation.

Selon une réalisation, on utilise le dispositif immersif pour procurer du bien-être et/ou en accompagnement ou en complément thérapeutique.

Par « thérapeutique », on entend un dispositif qui est utilisé comme thérapie ou comme accompagnant la mise en place d'une thérapie. Le dispositif immersif thérapeutique participe au bien-être d'un utilisateur qui suit une thérapie.

Par « bien-être », on entend sensation agréable.

### BREVE DESCRIPTION DES DESSINS

Des modes de réalisation de l'invention seront décrits ci-dessous par référence aux dessins, décrits brièvement ci-dessous :
[Fig. 1] représente une vue de profil d'un utilisateur équipé d'un dispositif de traitement de la maladie d'Alzheimer selon un mode de réalisation.

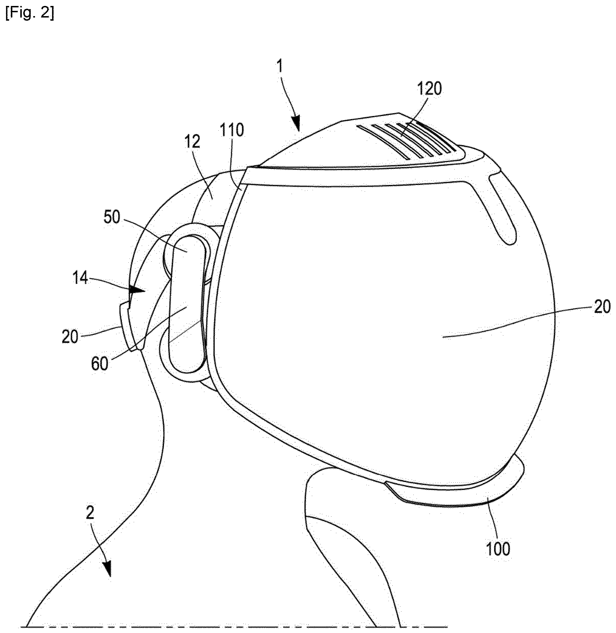

[Fig. 2] représente une vue en trois dimensions depuis un point de vue de côté et face à un utilisateur équipé d'un dispositif de traitement de la maladie d'Alzheimer selon le même mode de réalisation.
[Fig. 3] représente une vue en trois dimensions depuis un point de vue de côté et arrière d'un utilisateur équipé d'un dispositif de traitement de la maladie d'Alzheimer selon le même mode de réalisation.

Sur les dessins, des références identiques désignent des objets identiques ou similaires.

### DESCRIPTION DETAILLEE

La description détaillée présente un mode de réalisation du dispositif immersif.

Le dispositif immersif 1 représenté sur les figures est en position d'utilisation sur un utilisateur 2.

Ledit dispositif comporte trois principales parties :
- Un élément de fixation 10,
- une visière 20,
- un lien 30 entre l'élément de fixation 10 et la visière 20.

Une quatrième partie 60, optionnelle, couvre les oreilles de l'utilisateur et peut diffuser le son d'une vidéo diffusée. Une autre option consiste à utiliser des haut-parleurs.

L'élément de fixation 10 est par exemple en PVC. Dans l'exemple présenté, l'élément de fixation forme un harnais comprenant une pluralité de bandes serrées sur le crane de l'utilisateur. Il comprend deux parties, comme cela sera détaillé ci-après : L'élément de fixation 10 comprend une première partie 12 qui vient se positionner sur le dessus du crâne de l'utilisateur. Cette première partie comprend une bande droite 12, en PVC ou en tissu, ajustable en longueur afin de régler en largeur et en hauteur le dispositif immersif 1. Cette bande droite ajustable 12 part d'un point d'assemblage situé près d'une tempe de l'utilisateur et s'étend jusqu'à un point d'assemblage situé près de la tempe opposée. Ces deux points sont des points d'assemblage 50 où viennent se fixer les extrémités des différentes parties 30, 60 du dispositif immersif 1.

Le point d'assemblage droit 50 est situé à proximité de la tempe droite de l'utilisateur 2. Un point d'assemblage gauche est situé à proximité de la tempe gauche de l'utilisateur 2. En variante, on pourrait utiliser tout type de système de réglage adapté.

L'élément de fixation 10 comprend une deuxième partie 14 qui vient se positionner sur l'arrière de la tête de l'utilisateur 2. Cette deuxième partie 14 est constituée de deux bandes rigides 16 reliées par une roue de serrage 18 couplée à un mécanisme 19 permettant une prise d'appui sur la partie en dessous du crâne de l'utilisateur 2. La prise d'appui est plus basse que les deux points d'assemblage 50 grâce à l'orientation des deux bandes rigides 14 vers le bas lorsque ledit dispositif est en position d'utilisation.

La roue de serrage 18 et le mécanisme 19 relient les deux bandes rigides 16 et une action manuelle en rotation est transformée par la roue de serrage 18 en mouvement de translation par le mécanisme 19. En inversant le sens de rotation de la roue de serrage 18, on inverse le sens du mouvement de translation appliqué par le mécanisme 19 sur les deux bandes rigides 16. Ainsi, on peut ajuster le serrage de la deuxième partie 14 sur la largeur et la profondeur de la tête de l'utilisateur 2. En variante, on pourrait utiliser tout autre dispositif de serrage adapté.

Sur chacun des points d'assemblage gauche et droit 50, la quatrième partie 60 comprend des écouteurs audio placés pour couvrir chaque oreille de l'utilisateur 2 lorsque le dispositif immersif 1 est en position d'utilisation sur la tête de l'utilisateur 2. Les écouteurs audio sont reliés au système de diffusion de vidéos et sont aptes à diffuser le son associé à une vidéo.

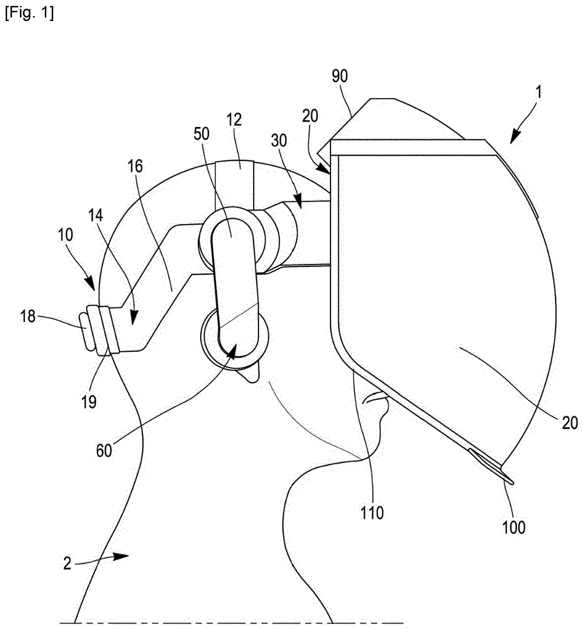

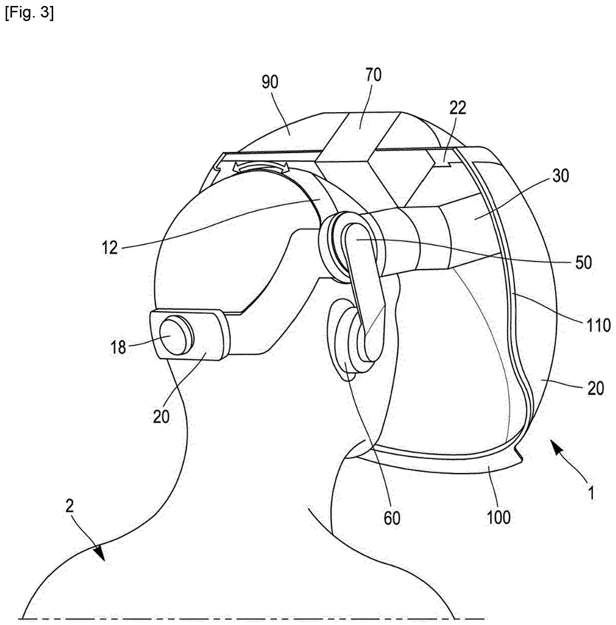

A partir des deux points d'assemblage symétrique gauche et droit 50, un lien 30 permet de fixer une visière 20 à distance du visage de l'utilisateur 2 lorsque le dispositif immersif 1 est en position d'utilisation. Le lien 30 est par exemple en PVC. Comme visible sur les figures 1 et 3, le lien 30 est orienté de manière à s'écarter latéralement par rapport au plan vertical médian de la tête de l'utilisateur. Ainsi, les liens 30 présentent une géométrie conique s'élargissant depuis la tête de l'utilisateur 2 vers l'avant. La visière 20 sert d'écran. La visière est par exemple en polycarbonate. Grâce au polycarbonate, on obtient une visière dotée d'une surface bien lisse, très résistante aux chocs, et sans effet sur la santé en l'absence de contact avec certains agents chimiques, et qui peut être transparente ou opaque, par exemple blanche. Ainsi, la visière est plus légère et simple à fabriquer qu'un verre sphérique.

**En position** d'utilisation, la visière 20 s'étend depuis au-dessus du haut du front jusqu'au niveau face au menton de l'utilisateur 2 pour former une portion de sphère globalement équidistante du visage de l'utilisateur 2, à environ 20 cm par exemple. Comme visible sur la figure 1, la portion de sphère, d'étendue inférieure à une demie-sphère, s'étend, en direction de l'arrière de la tête, jusque derrière le plan vertical des yeux. La visière 20 présente ainsi un pourtour périphérique qui, en position d'utilisation, est distant du visage de l'utilisateur. L'entier champ visuel de l'utilisateur est intercepté par la visière 20. Toutefois, l'angle solide occupé par la visière 20 ne va pas au-delà, ou va peu au-delà du champ visuel de l'utilisateur.

Il peut y avoir, en fonction du mode de réalisation, et selon la partie du visage concernée, entre 12 cm et 20 cm entre le visage de l'utilisateur 2 et la visière 20. Ainsi, le dispositif immersif 1 n'a aucun point de contact avec le visage en position d'utilisation.

La visière 20 comprend un système de diffusion de vidéos. Au niveau de la visière 20 située face au front, au-dessus des yeux de l'utilisateur 2, un support 70 pour projecteur permet de fixer un projecteur. Un projecteur est fixé au support 70 pour projecteur. Ainsi, le système de diffusion de vidéos comprend un projecteur. Par conséquent, la visière 20 comprend également un projecteur. Le projecteur est adapté pour projeter une image sur la face intérieure 21 de l'écran. Dans le mode de réalisation représenté, il s'agit d'un pico-projecteur LBS (« Laser Beam Splitting », qui signifie « division du faisceau laser ») ou DLP (« Digital Light Processing », qui signifie «traitement numérique de la lumière ») pour projeter une image, et en particulier une succession d'images formant une vidéo. Chaque image a été traitée par ordinateur à l'aide d'un algorithme afin de corriger certaines déformations d'affichage.

Le pico-projecteur LBS permet d'avoir une image toujours nette, même en cas de projection en biais. Il reste néanmoins à corriger les déformations d'affichage dûes à la courbure de l'écran.

Une optique sur mesure interagit avec le faisceau lumineux émis par le pico-projecteur LBS pour participer à la correction de l'image afin de reproduire une image nette et non déformée de l'image d'origine sur la surface galbée, par exemple sphérique, interne de la visière 20. Par image fidèle, on entend une image perçue par l'utilisateur 2 comme globalement similaire à l'image enregistrée d'origine. Le système de diffusion de séquence vidéos comprend une mémoire ou un système d'accès à une mémoire, dans laquelle sont stockées des séquences vidéos. Ces séquences ont par exemple préalablement été classées pour répondre à une ou plusieurs émotions. Selon un exemple de réalisation, les séquences vidéos sont chacune associées à une notation provenant d'une évaluation préalable. De plus ou en alternative, les séquences vidéos peuvent être associées à un historique de diffusion. Un compteur associé à une vidéo peut donc être incrémenté à chaque nouvelle diffusion de la vidéo. L'historique de diffusion, peut être associé à chaque utilisateur donné. Cette dernière réalisation nécessite que, au moment de l'utilisation du dispositif immersif, celui-ci comprenne un moyen d'identification de l'utilisateur. Cette identification peut être renseignée de manière manuelle, par mesure biométrique ou autre.

En plus du système de diffusion de vidéos, un système de reconnaissance d'émotions permet de détecter en temps réel les émotions de l'utilisateur grâce à des capteurs reliés à un calculateur capable d'interpréter les signaux reçus en une émotion, éventuellement pondérée, ou plusieurs émotions pondérées. Les émotions détectées peuvent être par exemple choisies dans la liste suivante : la joie, la tranquillité, la tristesse, la colère, la peur, la surprise, l'ennui, le mépris, la fatigue et le dégoût.

Ainsi, le système de reconnaissance d'émotions comprend, comme exemple des capteurs mentionnés ci-dessus, une caméra 22, de type webcam, positionnée pour filmer le visage de l'utilisateur 2. Par exemple, la visière 20 comprend cette caméra 22. Cette caméra 22 est reliée au système de diffusion de séquences vidéo. Le système de reconnaissance d'émotions analyse et extrait une liste d'émotions pondérées en temps réel en fonction de l'image captée par la caméra 22.

De tels systèmes peuvent comprendre d'autres capteurs couplés ou non entre eux : sudation, température, activité électrodermale, pouls, détection des pupilles, etc...

Si la pondération d'une des émotions dépasse un certain seuil, ou si la séquence vidéo en cours de diffusion se termine, le système de diffusion de vidéos sélectionne une catégorie de séquences vidéo disponibles en fonction de l'émotion captée.

Pour choisir une séquence vidéo parmi celles disponibles dans une catégorie correspondant à l'émotion ou à la combinaison d'émotions détectée, plusieurs critères peuvent être utilisés, seuls ou en combinaison : un choix aléatoire, la fréquence de diffusion de la séquence vidéo, une séquence vidéo pas encore diffusée à cet utilisateur, une vidéo très bien évaluée, etc.

Une fois la vidéo sélectionnée dans la catégorie correspondant à l'émotion ou à la combinaison d'émotions détectée, elle est diffusée par le projecteur sur la face intérieure 21 de la visière 20 qui sert d'écran, accompagnée éventuellement d'un son enregistré.

Les séquences vidéo s'enchainent. On peut programmer automatiquement des pauses et/ou un arrêt final dans cet enchainement de séquences vidéo.

En complément, le dispositif immersif comprend un système d'observation adapté pour capter les réactions de l'utilisateur. Pour le système d'observation, on peut utiliser les mêmes capteurs ou caméras que celles du système de reconnaissance d'émotions, ce qui permet une économie de matériel. Les données mesurées peuvent être enregistrées en vue d'être analysées ultérieurement. Le système d'observation peut être adapté pour diffuser les réactions de l'utilisateur à un tiers, notamment un observateur souhaitant connaître les réactions de l'utilisateur à l'expérience immersive. Cette diffusion peut par exemple se faire par une communication d'informations filaire ou sans fil. Les données captées et enregistrées (par exemple, le visage de l'utilisateur filmé) peuvent être affichées sur un écran de contrôle de l'observateur. Ainsi, celui-ci peut agir au cas où l'observation de l'utilisateur le nécessite.

Ainsi, selon un exemple de réalisation, on a recours à un système qui comprend le dispositif immersif 1 tel que présenté ci-dessus et un poste de contrôle. Le poste de contrôle est accessible à un observateur qui accède ainsi aux informations relatives à l'utilisation du dispositif. Le poste de contrôle peut ainsi être utilisé pour afficher des signaux issus des capteurs du dispositif immersif 1, ainsi que le contenu multimédia diffusé, le cas échéant simultanément. Ces données peuvent être affichées en temps réel, simultanément à l'utilisation, ou postérieurement. Le poste de contrôle peut également être utilisé pour sélectionner, ou aider à sélectionner, une séquence multimédia à diffuser. Le cas échéant, le poste de contrôle peut être associé à plusieurs dispositifs immersifs 1 utilisés par différents utilisateurs.

L'alimentation électrique dudit dispositif immersif 1 peut se faire par un fil électrique non représenté relié à une alimentation extérieure. Dans le but d'alléger au maximum ledit dispositif immersif 1, de bénéficier d'une longue autonomie et d'éviter des interruptions brutales néfastes à une thérapie, une alimentation filaire extérieure non représentée est avantageuse.

Le dispositif immersif 1 comprend un système de préhension. Le système de préhension comprend par exemple deux éléments :
- Le haut de la visière 20 présente un rebord plat de préhension 90 aligné, de chaque côté du support pour projecteur 70, avec la face arrière dudit support 70 ;
- une bavette de protection et de préhension 100 est fixée sur le bord de la visière 20 en face du menton de l'utilisateur 2.

Ainsi, avec ses deux mains, un opérateur peut positionner et retirer le dispositif immersif 1 aisément.

Une bande de renfort 110 entoure la visière 20 afin de protéger ladite visière 20 d'éventuels chocs en utilisation ou en manipulation.

Des ouvertures 120 sur le haut de la visière 20, au niveau où le projecteur est positionné, permettent d'évacuer la chaleur générée par le fonctionnement du projecteur.

L'invention n'est pas limitée aux modes de réalisations présentées et d'autres modes de réalisation apparaîtront clairement à l'homme du métier.

Notamment en cas de projecteur plus volumineux, le support de projecteur 70 peut être placé à l'extérieur de la visière, au-dessus de la tête, incliné vers la visière, et le projecteur peut être positionné sur ledit support de projecteur 70 de façon à diriger le flux sur la face interne de la visière. Ainsi, on dispose d'assez d'espace pour placer des projecteurs plus volumineux.

Selon un mode de réalisation n'étant pas couvert par les revendications, vidéoprojecteur et l'écran passif peuvent être remplacés par un écran actif composé de LED.

Le PVC et/ou le polycarbonate utilisés dans le mode de réalisation présenté peuvent être remplacés par des matériaux innovants sur lesquels les bactéries et les virus ont du mal à s'accrocher.

L'opacité de la visière peut également être commandée par le système de diffusion de vidéos afin d'alterner des projections de séquences vidéo et un accès visuel à l'environnement extérieur. Ainsi, à la fin d'une séance de thérapie réalisée à l'aide dudit dispositif, l'utilisateur a accès à une vision sur la réalité extérieure sans avoir à attendre qu'on lui retire le dispositif immersif 1.

Pour accéder rapidement à une vision sur la réalité extérieure, un autre mode réalisation plus simple techniquement comprend une visière 20 dotée d'un système d'attache réversible permettant à l'utilisateur de retirer manuellement la visière 20 facilement du dispositif immersif 1. On prévoit un système d'attache réversible notamment entre la visière 20 et le lien 30.

Le dispositif immersif 1 peut également comprendre un micro adapté pour capter les sons émis par l'utilisateur. Ces sons peuvent être enregistrés et transmis au poste de contrôle. Le poste de contrôle peut comprendre un micro adapté pour capter les sons émis par l'observateur, et pour transmettre ces sons au dispositif immersif. Ainsi, par exemple, un dialogue peut s'engager, si besoin, entre l'utilisateur et l'observateur.

En fournissant un dispositif immersif comprenant une visière occupant une portion de sphère interceptant le regard de l'utilisateur, et espacée du visage, et un harnais de fixation sur la tête, on rend le dispositif immersif le moins invasif possible pour éviter qu'il ne soit source d'angoisse, de stress et de fatigue pour l'utilisateur. En effet, aucune contrainte mécanique n'est appliquée sur le visage. Le système est compatible pour les utilisateurs portant des lunettes. L'espace entre le visage et le dispositif peut ainsi être utilisé à bon escient pour installer des équipements, par exemple une ou plusieurs caméras pouvant filmer le visage du patient. La lumière ambiante susceptible d'entrer entre le visage et le bord périphérique de la visière permet de disposer d'un degré de luminosité suffisant pour filmer le visage. Cet espace permet également la circulation d'un flux d'air pour la ventilation de l'utilisateur. Enfin, du point de vue de l'hygiène, la visière n'est pas ou peu contaminée par l'utilisateur, et peut ensuite être rapidement utilisée pour un autre utilisateur, moyennant, le cas échéant, le remplacement intermédiaire de l'élément de fixation.

## Revendications

1. Dispositif immersif thérapeutique ou de bien-être (1) qui comprend :
- un élément de fixation (10) pour fixer le dispositif sur la tête d'un utilisateur (2),
- une visière (20) qui sert d'écran passif, et s'étendant depuis au-dessus du haut du front jusqu'au niveau face au menton, et galbée pour être à distance du visage en position d'utilisation et pour occuper le champ visuel de l'utilisateur (2),
- un lien (30) entre l'élément de fixation (10) et la visière (20),
- un système de reconnaissance d'émotions apte à détecter des émotions de l'utilisateur, le système de reconnaissance d'émotions comprenant au moins un capteur et un calculateur analysant un ou plusieurs signaux du capteur pour indiquer une émotion choisie parmi la joie, la tranquillité, la tristesse, la colère, la peur, la surprise, l'ennui, le mépris, la fatigue et le dégoût,
- un système de diffusion de séquences vidéo, une séquence vidéo comprenant une suite d'images diffusées, le système de diffusion étant adapté pour sélectionner une séquence vidéo classée par rapport à au moins une émotion de l'utilisateur communiquée par le calculateur du système de détection, et comprenant un vidéoprojecteur fixé au-dessus de la tête de l'utilisateur (2) en position d'utilisation pour diffuser la séquence vidéo sur l'écran passif, et
- un système d'observation pour capter, diffuser et/ou enregistrer les réactions de l'utilisateur (2).

2. Dispositif immersif thérapeutique ou de bien-être (1) selon la revendication 1, comprenant en outre l'une et/ou l'autre des caractéristiques suivantes :
- le système d'observation comprend une caméra (22), orientée vers le visage de l'utilisateur (2) ;
- l'élément de fixation est ajustable.

3. Dispositif immersif thérapeutique ou de bien-être (1) selon la revendication 1 ou 2, dans lequel le système de diffusion de séquences vidéo comprend un support permettant de supporter le vidéoprojecteur de façon réversible.

4. Dispositif immersif thérapeutique ou de bien-être (1) selon l'une des revendications 1 à 3, dans lequel ledit dispositif immersif thérapeutique ou de bien-être (1) est doté dans sa partie basse d'une bavette (100).

5. Dispositif immersif thérapeutique ou de bien-être (1) selon l'une quelconque des revendications 1 à 4, dans lequel le dispositif est adapté pour déconnecter l'utilisateur de son environnement et lui donner l'impression d'être plongé dans un environnement différent en sollicitant ses sens : la vue et l'ouïe.

6. Dispositif immersif thérapeutique ou de bien-être (1) selon l'une quelconque des revendications 1 à 5, dans lequel les séquences vidéo comprennent chacune une suite d'images diffusées dont le contenu a été testé comme adapté à une ou plusieurs émotions humaines dans un but de générer de la joie, de la tranquillité ou de stimuler les fonctions cognitives et la concentration du cerveau de l'utilisateur.

7. Dispositif immersif thérapeutique ou de bien-être (1) selon l'une quelconque des revendications 1 à 6, dans lequel le dispositif immersif (1) ne présente aucun point de contact avec le visage en position d'utilisation.

8. Dispositif immersif thérapeutique ou de bien-être (1) selon l'une quelconque des revendications 1 à 7, dans lequel le lien (30) rigide est évasé vers l'extérieur dans le sens allant de l'élément de fixation (10) vers la visière (20).

9. Dispositif immersif thérapeutique ou de bien-être (1) selon l'une quelconque des revendications 1 à 8, dans lequel la visière (20) présente un pourtour périphérique distant du visage de l'utilisateur en position d'utilisation.

10. Système comprenant un poste de contrôle et au moins un voire plusieurs, dispositifs immersifs thérapeutiques ou de bien-être (1) selon l'une quelconque des revendications 1 à 9.

11. Utilisation du dispositif immersif thérapeutique ou de bien-être (1) selon l'une des revendications 1 à 9, pour procurer du bien-être et/ou en accompagnement ou en complément thérapeutique.

## Patentansprüche

1. Therapeutische oder Wellness-Immersionsvorrichtung (1), umfassend:
- ein Befestigungselement (10) zum Befestigen der Vorrichtung am Kopf eines Benutzers (2),
- ein Visier (20), das als passive Projektionsfläche dient und sich vom Bereich oberhalb der Stirn bis auf Höhe des Kinns erstreckt und so gekrümmt ist, dass es sich in Gebrauchsposition in einem Abstand vom Gesicht befindet und das Sichtfeld des Benutzers (2) einnimmt,
- eine Verbindung (30) zwischen dem Befestigungselement (10) und dem Visier (20),
- ein Emotionserkennungssystem, das geeignet ist, Emotionen des Benutzers zu erkennen, wobei das Emotionserkennungssystem mindestens einen Sensor und eine Recheneinheit umfasst, die ein oder mehrere Signale des Sensors analysiert, um eine Emotion anzugeben, die aus Freude, Ruhe, Traurigkeit, Wut, Angst, Überraschung, Langeweile, Verachtung, Müdigkeit und Ekel ausgewählt ist,
- ein System zur Wiedergabe von Videosequenzen, wobei eine Videosequenz eine Folge wiedergegebener Bilder umfasst, wobei das Wiedergabesystem dazu ausgelegt ist, eine Videosequenz auszuwählen, die in Bezug auf mindestens eine Emotion des Benutzers klassifiziert ist, die von der Recheneinheit des Erkennungssystems übermittelt wird, und einen Videoprojektor umfasst, der in Gebrauchsposition oberhalb des Kopfes des Benutzers (2) befestigt ist, um die Videosequenz auf der passiven Projektionsfläche wiederzugeben, und
- ein Beobachtungssystem zum Erfassen, Übertragen und/oder Aufzeichnen der Reaktionen des Benutzers (2).

2. Therapeutische oder Wellness-Immersionsvorrichtung (1) nach Anspruch 1, ferner umfassend eines oder beide der folgenden Merkmale:
- das Beobachtungssystem umfasst eine Kamera (22), die auf das Gesicht des Benutzers (2) gerichtet ist;
- das Befestigungselement ist verstellbar.

3. Therapeutische oder Wellness-Immersionsvorrichtung (1) nach Anspruch 1 oder 2, wobei das System zur Wiedergabe von Videosequenzen eine Halterung umfasst, die den Videoprojektor lösbar hält.

4. Therapeutische oder Wellness-Immersionsvorrichtung (1) nach einem der Ansprüche 1 bis 3, wobei die therapeutische oder Wellness-Immersionsvorrichtung (1) in ihrem unteren Bereich mit einer Schürze (100) versehen ist.

5. Therapeutische oder Wellness-Immersionsvorrichtung (1) nach einem der Ansprüche 1 bis 4, wobei die Vorrichtung dazu ausgelegt ist, den Benutzer von seiner Umgebung zu entkoppeln und ihm den Eindruck zu vermitteln, in eine andere Umgebung eingetaucht zu sein, indem seine Sinne - Sehen und Hören - angesprochen werden.

6. Therapeutische oder Wellness-Immersionsvorrichtung (1) nach einem der Ansprüche 1 bis 5, wobei die Videosequenzen jeweils eine Folge von ausgestrahlten Bildern umfassen, deren Inhalt als für eine oder mehrere menschliche Emotionen geeignet getestet wurde, um Freude oder Ruhe zu erzeugen oder die kognitiven Funktionen und die Konzentration des Gehirns des Benutzers zu stimulieren.

7. Therapeutische oder Wellness-Immersionsvorrichtung (1) nach einem der Ansprüche 1 bis 6, wobei die Immersionsvorrichtung (1) in Gebrauchsstellung keine Kontaktstelle mit dem Gesicht aufweist.

8. Therapeutische oder Wellness-Immersionsvorrichtung (1) nach einem der Ansprüche 1 bis 7, wobei das starre Verbindungselement (30) in der Richtung vom Befestigungselement (10) zum Visier (20) nach außen aufgeweitet ist.

9. Therapeutische oder Wellness-Immersionsvorrichtung (1) nach einem der Ansprüche 1 bis 8, wobei das Visier (20) in Gebrauchsstellung einen vom Gesicht des Benutzers beabstandeten Umfangsrand aufweist.

10. System, umfassend eine Steuereinheit und mindestens eine, gegebenenfalls mehrere therapeutische oder Wellness-Immersionsvorrichtungen (1) nach einem der Ansprüche 1 bis 9.

11. Verwendung der therapeutischen oder Wellness-Immersionsvorrichtung (1) nach einem der Ansprüche 1 bis 9 zur Förderung des Wohlbefindens und/oder als therapeutische Begleitung oder Ergänzung.

## Claims

1. Therapeutic or well-being immersive device (1) which comprises:
- a fixing element (10) for fixing the device on the head of a user (2),
- a visor (20) which serves as a passive screen, and extending from above the top of the forehead to the level facing the chin, and curved so as to be at a distance from the face in the position of use and to occupy the visual field of the user (2),
- a link (30) between the fixing element (10) and the visor (20),
- an emotion recognition system capable of detecting emotions of the user, the emotion recognition system comprising at least one sensor and a computer analyzing one or more signals from the sensor to indicate an emotion chosen from joy, tranquility, sadness, anger, fear, surprise, boredom, contempt, fatigue and disgust,
- a system for broadcasting video sequences, a video sequence comprising a series of broadcast images, the broadcasting system being adapted to select a video sequence classified with respect to at least one emotion of the user communicated by the computer of the detection system, and comprising a video projector fixed above the head of the user (2) in the position of use to broadcast the video sequence on the passive screen, and
- an observation system for capturing, broadcasting and/or recording the reactions of the user (2).

2. Therapeutic or well-being immersive device (1) according to claim 1, further comprising one and/or the other of the following features:
- the observation system comprises a camera (22), oriented towards the face of the user (2);
- the fixing element is adjustable.

3. Therapeutic or well-being immersive device (1) according to claim 1 or 2, in which the system for broadcasting video sequences comprises a support making it possible to support the video projector in a reversible manner.

4. Therapeutic or well-being immersive device (1) according to one of claims 1 to 3, in which said therapeutic or well-being immersive device (1) is equipped in its lower part with a flap (100).

5. Therapeutic or well-being immersive device (1) according to any one of claims 1 to 4, in which the device is adapted to disconnect the user from his environment and give him the impression of being immersed in a different environment by appealing to his senses: sight and hearing.

6. Therapeutic or well-being immersive device (1) according to any one of claims 1 to 5, in which the video sequences each comprise a series of broadcast images whose content has been tested as adapted to one or more human emotions with the aim of generating joy, tranquility or to stimulate the cognitive functions and concentration of the user's brain.

7. Therapeutic or well-being immersive device (1) according to any one of claims 1 to 6, in which the immersive device (1) has no point of contact with the face in the position of use.

8. Therapeutic or well-being immersive device (1) according to any one of claims 1 to 7, in which the rigid link (30) is flared outwards in the direction going from the fixing element (10) towards the visor (20).

9. Therapeutic or well-being immersive device (1) according to any one of claims 1 to 8, in which the visor (20) has a peripheral periphery remote from the face of the user in the position of use.

10. System comprising a control station and at least one, or even several, therapeutic or well-being immersive devices (1) according to any one of claims 1 to 9.

11. Use of the therapeutic or well-being immersive device (1) according to one of claims 1 to 9, to provide well-being and/or as a therapeutic accompaniment or supplement.
